Europäisches Patentamt

European Patent Office

Office européen des brevets

(11) Publication number: 0 325 360
A1

# EUROPEAN PATENT APPLICATION

(21) Application number: 89300235.2

(22) Date of filing: 11.01.89

(51) Int. Cl.⁴: A61F 15/00

(30) Priority: 11.01.88 US 142832

(43) Date of publication of application:
26.07.89 Bulletin 89/30

(84) Designated Contracting States:
AT BE CH DE ES FR GB GR IT LI LU NL SE

(71) Applicant: Behringer, John Walter
143 Main Street
North Kingstown§Rhode Island 02852(US)

(72) Inventor: Behringer, John Walter
143 Main Street
North Kingstown§Rhode Island 02852(US)

(74) Representative: Leale, Robin George et al
FRANK B. DEHN & CO. Imperial House 15-19
Kingsway
London WC2B 6UZ(GB)

(54) Dispenser for packaged bandages and the like.

(57) An apparatus is provided for the storage and
dispensing of packaged adhesive bandages and
similarly-shaped flat packaged units. The dispenser
includes a drive unit base (12), which may be power-
ed either manually or electrically. A cartridge (14) is
removably mountable to the top of the base. The
cartridge holds a stack of packaged adhesive ban-
dages (56) on a floor (46) in which is defined an
elongate slot (48). A weight (58) is provided to exert
downward pressure against the bandages toward the
cartridge floor. A roller (18) with an elastic follower
finger (32) attached to the roller is mounted within
the housing and aligned with the slot in the floor.
When the roller is rotated the follower finger fric-
tionally engages the lowermost bandage (16) in the
stack, urging it through an exit portal (50) at one end
of the cartridge. As the bandage is propelled through
the portal, it displaces a resilient flap (54) which
covers the portal.

FIG. 1

## Dispenser for packaged bandages and the like

This invention relates to a dispenser which can be used to dispense single flat packaged units such as packaged bandages.

Packaged adhesive bandages are typically sold packed in a metal or cardboard package or canister. Each adhesive bandage is individually wrapped to maintain sterility. To use one of these bandages the user must open the canister and either pluck a single bandage therefrom or empty some or all of the contents of the canister, make a selection, and return the remaining bandages to the package.

While the need for efficient dispensing of adhesive bandages may be great in the context of families with small children, it is all the more pronounced in clinical settings. In doctor's offices, emergency rooms and medical laboratories, adhesive bandages are used often, especially to cover the small wounds created when blood is drawn for diagnostic testing.

According to the present invention there is provided apparatus for storing and dispensing flat package units, comprising:
a base;
package unit engagement means rotatably mounted within said base;
a cartridge mountable to said base, said cartridge defining a chamber therewithin for the storage of flat package units;
said chamber having a floor with a longitudinally disposed elongate slot defined therein alignable with said engagement means;
means for biasing toward said floor a plurality of flat package units stacked on said floor;
means associated with said base for rotatably driving said engagement means; and
an exit portal defined in said cartridge adjacent to one end of said chamber floor,
whereby rotation of said engagement means causes said engagement means to frictionally engage a lowermost package unit from said plurality in said chamber by way of said slot and to urge said package unit toward and through said portal.

In a preferred form of the invention packaged bandages or other flat package units are stacked in a chamber inside a cartridge and are held in place by a weight or other biasing means. They are stacked on a chamber floor which includes a centrally disposed longitudinally aligned slot. An exit portal is provided at the bottom of one end of the product cartridge, and is provided with a restraining flap to restrain all but one bandage, to assure that only one bandage at a time is dispensed. The complete dispenser is assembled by inserting the cartridge into the drive unit base. The drive unit base includes a roller or wheel whose axis is aligned horizontally, perpendicular to the slot defined in the chamber floor, so that a projection or flexible finger attached to the roller can, as the roller is rotated, frictionally engage the lowermost bandage in the cartridge through the slot. In this manner the lowermost bandage is urged against the restraining flap at the end of the slot and propelled out of the exit portal of the dispenser.

In alternative embodiments the roller may be powered manually by a simple crank arrangement or electrically by a motor drive.

The cartridge may be either refillable or replaceable, and may be provided with insertable inner sleeves to accommodate different size bandages.

Two embodiments of the invention will now be described by way of example and with reference to the accompanying drawings, in which:-

Figure 1 is a general perspective view of a dispenser embodying the present invention;

Figure 2 is an exploded view of the dispenser shown in Figure 1. with the product cartridge shown disengaged from the base unit;

Figure 3 is a cross-sectional view taken along line 3-3 of Figure 1 illustrating the operation of the dispenser;

Figure 4 is a cross-sectional view taken along line 4-4 of Figure 1 illustrating the operation of the dispenser;

Figure 5 is a cross-sectional view taken along line 5-5 of Figure 4; and

Figure 6 is an exploded view of a second embodiment.

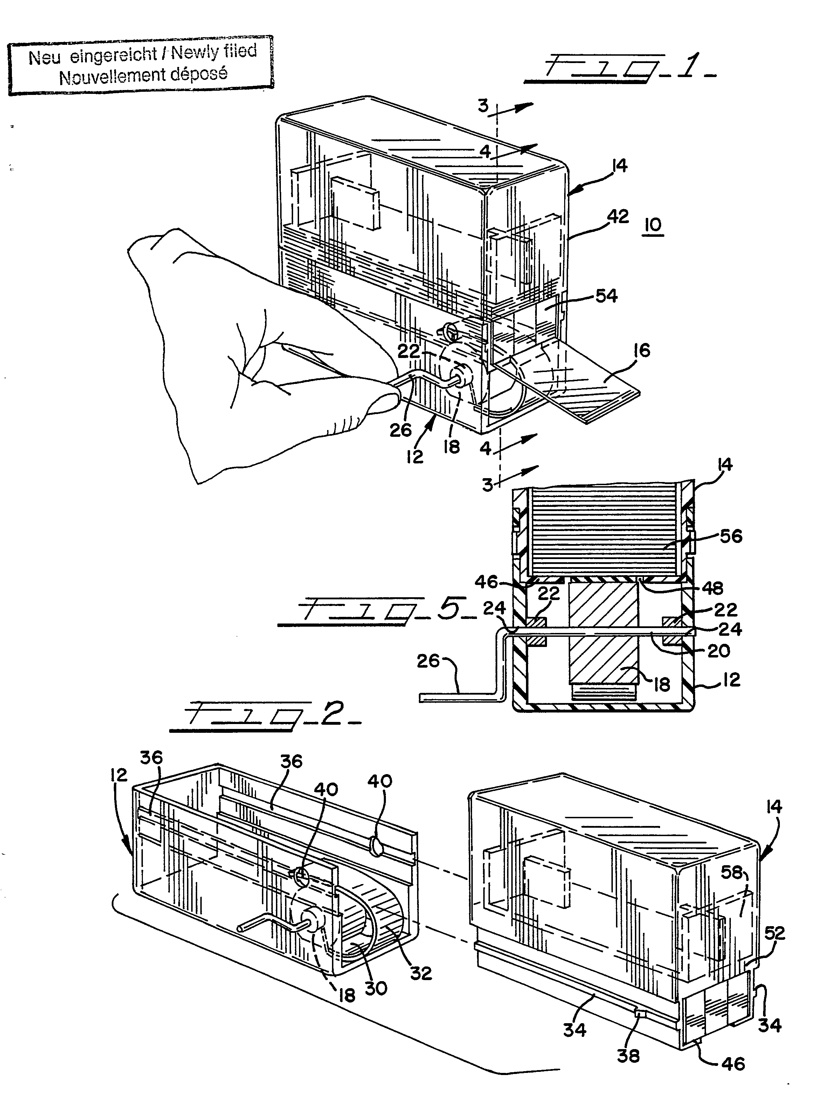

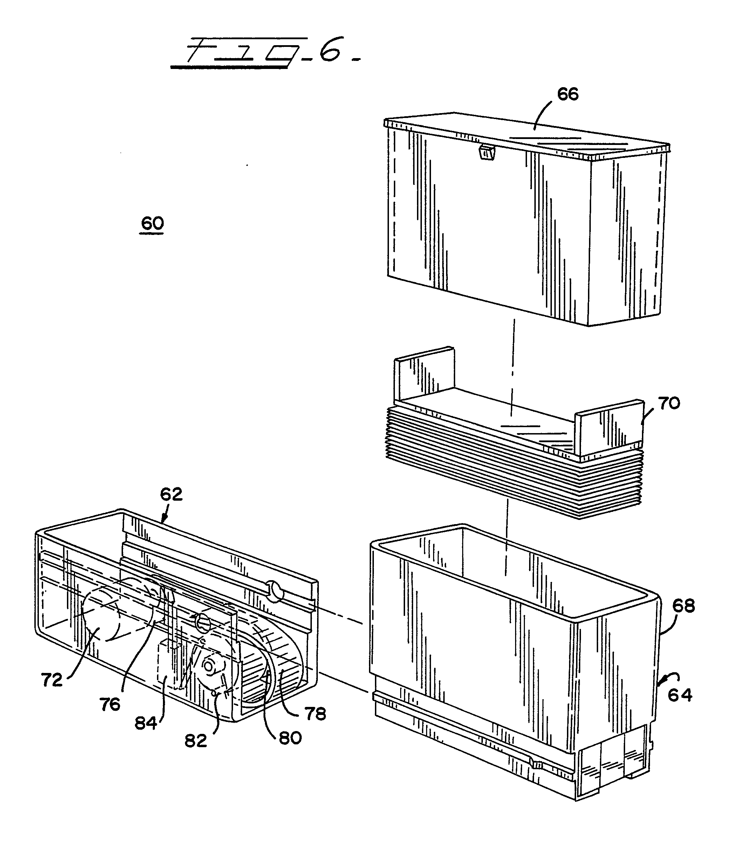

Referring now to Figure 1 of the drawings, a bandage dispenser embodying the present invention is generally indicated by reference numeral 10. In this embodiment the device includes a drive unit base 12 connected to a product cartridge 14. Figure 1 is a general perspective view of the embodiment, and shows the dispenser in use dispensing one of the packaged adhesive bandages 16 stored in cartridge 14. Illustrated in phantom within base 12 is a roller 18, which is rotatably mounted to base 12 by means of an axle 20 which has journals 22 fixed thereto. Bearing sockets 24 are defined in the walls of base 12 to receive axle 20. Roller 18 is fixed to axle 20 and is located substantially midway between the two sides of base 12. Extending from axle 20 through the wall of base 12 is a crank 26. The arrangement of these components may be readily appreciated by reference to Figure 5.

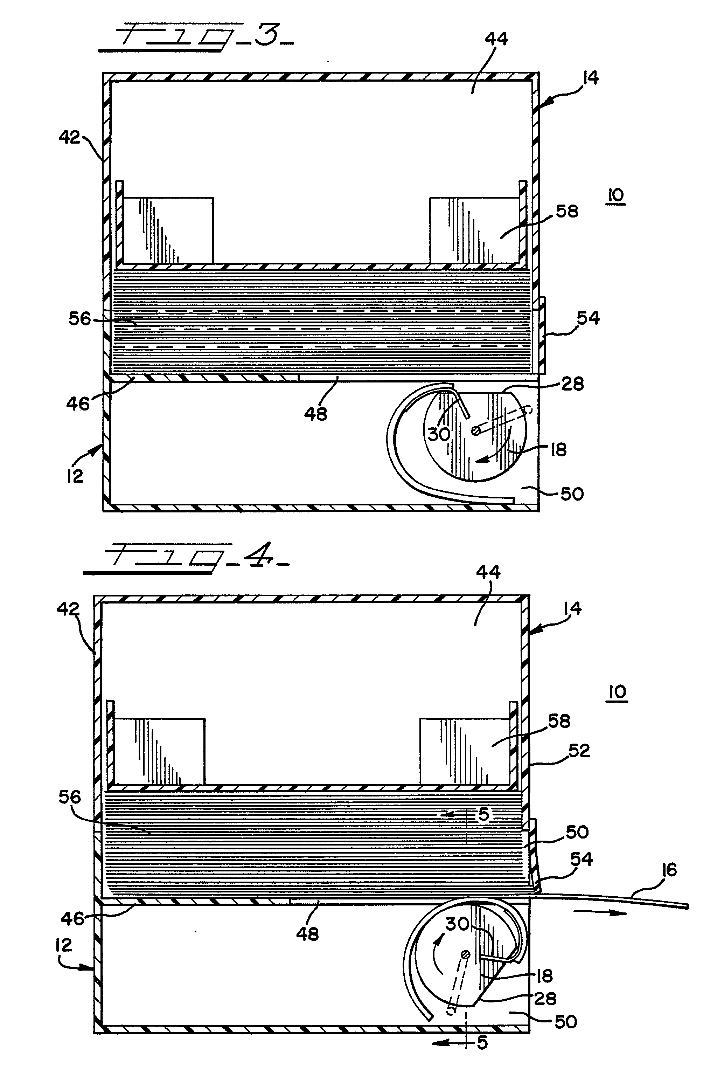

Roller 18 is substantially circular-cylindrical in shape, except that the cylinder is truncated along a plane parallel to the axis of the roller, thereby defining a planar face 28 (Figures 3 and 4) on the roller.

Fixed to roller 8 at face 28 is an extended anchor 30. As shown, in the embodiment anchor 30 is formed from a resilient, semi-rigid material such as plastics or spring steel. Affixed to anchor 30 is a flexible follower finger 32. Finger 32 in this embodiment is made of a suitable elastic material such as natural or synthetic rubber.

Cartridge 14 slidably engages with drive unit base 12 by slidable mating of horizontal tracks 34 on cartridge 14 with respective associated channels 36 in the drive unit base. To complete the assembly of the unit, projecting lugs 38 on tracks 34 engage sockets 40 in channels 36. The truncation of roller 18 provides clearance for the cartridge to slide past roller 18 when face 28 is in the uppermost horizontal position, as shown in Figure 3.

As illustrated, in this embodiment cartridge 14 includes a cartridge housing 42 in which is defined a cartridge chamber 44. Cartridge 14 also includes a horizontal cartridge chamber floor 46, with an elongate slot 48 defined therein. Slot 48 is disposed substantially parallel to the sides of the chamber 44 and located substantially medially thereof. Slot 48 extends from a point intermediate the ends of chamber 44 to the front end of the chamber, where the slot is open ended at a portal 50 defined in front face 52 of cartridge 14. A flexible cover flap 54, made of a suitable elastic material, is attached along its top edge to face 52 above portal 50.

Individually packaged adhesive bandages 56 are stacked in cartridge chamber 44 on the chamber floor 46, with the lowermost bandage 16 resting on floor 46 and exposed to slot 48. A bias weight 58 is disposed within chamber 44, resting on top of stack 56 to maintain the stack in orderly arrangement. It is contemplated that in alternative embodiments other suitable biasing means may be used to maintain this arrangement, such as a spring to exert a downward bias against the stack of bandages 56.

Roller 18, finger anchor 30 and finger 32 are aligned with slot 48 so that when crank 6 is turned in the clokwise direction, finger 32 frictionally engages the lowermost bandage 16 through slot 48, as shown in Figures 3 and 4. As the roller 18 makes a complete rotation, bandage 16 is urged through portal 50, outwardly displacing cover flap 54, and propelled out of the dispenser as shown in Figure 4 and also in Figure 1. Elastic cover flap 54 serves to restrain all of the bandages other than the lowermost bandage 16, which is the only one frictionally engaged by finger 32.

Although finger 32 is showns as a flexible, elongate, elastic member, it is contemplated that alternative embodiments of this element may be used with similar effect, including a cam of unitary construction having a projection to frictionally engage lowermost bandage 16 through slot 48.

A second embodiment of the invention is shown in Figure 6, in exploded view, and is indicated generally by reference numeral 60. Dispenser 60 includes a motorized drive unit base 62 which is engageable with cartridge 64 in the same manner as in the previously described embodiment. An inner sleeve 66 is slidably fitted into an outer sleeve 68 of cartridge 64. Inner sleeve 66 may be variously dimensioned to accommodate various sizes of individually packaged adhesive bandages or similarly shaped product units. A bias weight 70 of suitable dimension is provided to fit within inner sleeve 66 and, as previously discussed, is used to maintain alignment of the stack of bandages withing the cartridge chamber, by gravity. Other suitable bias means could be employed.

Drive unit base 62 includes an electric motor 72 mounted within base 62. A roller 74, similar to roller 18 of the previous embodiment, is driven by motor 72 through a drive pulley 76, a drive belt 78 and a driven pulley 80 which is fixed to, and rotatable with, roller 74. A pin 82 is fixed to the side of roller 74 and projects outwardly therefrom, in parallel relation to the axis of the roller. A switch 84 is mounted to base 72 in operative proximity to roller 74, and aligned with projecting pin 82.

Electric power to motor 72 is provided through a circuit (not shown) which includes switch 84 in series with motor 72, so that when power is applied to motor 72, roller 74 is driven through one complete revolution, expelling one packaged bandage before switch 84 is tripped by pin 82 to open the electrical circuit, thus interrupting power to the motor.

It will thus be seen that the invention, at least in its preferred forms, provides a simple, attractive, functional dispenser for packaged adhesive bandages for storage and dispensing, or for similarly shaped flat product units; and furthermore provides such a dispenser for storage and dispensing of individual packaged adhesive bandages or like units, which may be either manually or electrically powered; and furthermore provides such a dispenser which is compact and easily stored.

It is to be clearly understood that there are no particular features of the foregoing specification, or of any claims appended hereto, which are at present regarded as being essential to the performance of the present invention, and that any one or more of such features or combinations thereof may therefore be included in, added to, omitted from or deleted from any of such claims if and when amended during the prosecution of this application or in the filing or prosecution of any divisional application based thereon. Furthermore the manner in which any of such features of the specification or claims are described or defined may be amended, broadened or otherwise modified in any manner which falls within the knowledge of a person skilled in the relevant art, for example so as to encompass, either implicitly or explicitly, equivalents or generalisations thereof.

## Claims

1. Apparatus for storing and dispensing flat package units, comprising:
a base;
package unit engagement means rotatably mounted within said base;
a cartridge mountable to said base, said cartridge defining a chamber therewithin for the storage of flat package units;
said chamber having a floor with a longitudinally disposed elongate slot defined therein alignable with said engagement means;
means for biasing toward said floor a plurality of flat package units stacked on said floor;
means associated with said base for rotatably driving said engagement means; and
an exit portal defined in said cartridge adjacent to one end of said chamber floor,
whereby rotation of said engagement means causes said engagement means to frictionally engage a lowermost package unit from said plurality in said chamber by way of said slot and to urge said package unit toward and through said portal.

2. Apparatus as claimed in claim 1, further comprising means associated with said exit portal to substantially restrain the exit of flat package units.

3. Apparatus as claimed in claim 2, wherein said restraining means comprises a resilient flap attached to said cartridge and covering said exit portal, whereby passage of said lowermost package unit through said portal partially displaces said resilient flap.

4. Apparatus as claimed in any of claims 1 to 3, wherein said slot is open-ended and the open end of said slot is substantially co-extensive with said portal.

5. Apparatus as claimed in any preceding claim, wherein said biasing means comprises a weight positionable on said plurality of packaged units.

6. Apparatus as claimed in any preceding claim wherein said engagement means comprises a substantially cylindrical roller having a flexible follower extending from said roller to frictionally engage said lowermost package unit by way of said slot.

7. Apparatus as claimed in claim 6, wherein said roller is a substantially circular cylinder truncated parallel to its central axis.

8. Apparatus as claimed in claim 6 or 7, wherein said follower is attached to a resilient extension anchor attached to said roller.

9. Apparatus as claimed in claim 8, wherein said resilient extension anchor maintains at least a portion of said follower spaced from said roller.

10. Apparatus as claimed in any preceding claim, wherein said driving means comprises a crank extending from an axle on which said engagement means is mounted.

11. Apparatus as claimed in any of claims 1 to 9, wherein said driving means comprises an electric motor mounted to said base in operative relation with said engagement means.

12. Apparatus as claimed in claim 11, further comprising means operatively associated with said engagement means to interrupt electric power to said electric motor.

13. Apparatus as claimed in claim 12, wherein said interrupting means comprises a pin projecting from said engagement means and a switch in electrical communication with said electric motor and in operative relation with said pin whereby rotation of said engagement means causes said pin to trip said switch, thereby interrupting power to said motor.

14. Apparatus as claimed in any preceding claim, wherein said cartridge comprises an elongate cartridge housing and an inner sleeve engageable with said housing, said inner sleeve being interiorly dimensioned to accommodate a predetermined size of flat package units.

FIG_1_

FIG_5_

FIG_2_

_Fig_3_

_Fig_4_

_FIG_.6.

60

66

70

68

64

62

72    76    84    82    80    78

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-2 645 543  (MANCINI)<br>* Column 1, line 43 - column 3, line 40; figures *<br>--- | 1,2,4, 10 | A 61 F   15/00 |
| A | US-A-4 043 549  (XEROX CORP.)<br>* Abstract; figures; column 2, line 53 - column 3, line 2 *<br>--- | 6-9 | |
| A | US-A-3 189 219  (HOLZWORTH)<br>--- | | |
| A | GB-A-1 089 640  (ETHICON)<br>----- | | |

TECHNICAL FIELDS
SEARCHED (Int. Cl.4)

A 61 F
B 65 D
B 65 H

The present search report has been drawn up for all claims

| Place of search | Date of completion of the search | Examiner |
|---|---|---|
| THE HAGUE | 19-04-1989 | STEENBAKKER J. |